# EUROPEAN PATENT SPECIFICATION

(11) **EP 1 732 515 B1**
(45) Date of publication and mention of the grant of the patent: **02.07.2008**
(21) Application number: 05722259.8
(22) Date of filing: 23.03.2005
(51) Int. Cl.: A61K 9/14, A61M 15/00, B65B 1/36, A61K 9/72

(54) **DRY POWDER PREPARATIONS FOR PRE-METERED DPI**
TROCKENPULVERZUBEREITUNGEN FÜR VORDOSIERTE TROCKENPULVERINHALATOREN
PREPARATIONS DE POUDRE SECHE POUR UN INHALATEUR A POUDRE SECHE DPI (PREDOSE)

(30) Priority: 29.03.2004 SE 0400844
(43) Date of publication of application: 20.12.2006
(73) Proprietor: Mederio AG, 6052 Hergiswil NW (CH)
(72) Inventor: NILSSON, Thomas, S-647 32 Mariefred (SE); FRIBERG, Claes, S-640 60 Åkers Styckebruk (SE); KAX, Lars, S-155 91 Nykvarn (SE); NIEMI, Alf, S-645 50 Strängnäs (SE); CALANDER, Sven, S-645 32 Strängnäs (SE)
(74) Representative: Stenborg, Anders Vilhelm
(86) International application number: PCT/SE2005/000418
(87) International publication number: WO 2005/092289

(56) References cited:
- EP-A1- 1 393 721
- WO-A1-00/71419
- WO-A1-01/27210
- WO-A1-02/18000
- WO-A1-02/24266
- WO-A1-03/066437
- WO-A1-03/086516
- US-A- 5 551 489
- US-A- 5 865 012
- US-A1- 2003 012 865
- US-A1- 2003 131 905

## Description

### TECHNICAL FIELD

The present invention relates to a preparation and a forming and loading of a dry powder medicament adapted for novel filling methods capable of producing metered medicament doses having improved performance intended for a pre-metered dry powder inhaler (DPI).

### BACKGROUND

The dosing of drugs is carried out in a number of different ways in the medical service today. Within health care there is a rapidly growing interest in the possibility of administering medication drugs as a powder directly to the airways and lungs of a patient by means of an inhaler in order to obtain an effective, quick and user-friendly delivery of such substances. Because the efficacy of inhaled doses often are much higher than e.g. orally administered capsules, the inhalation doses need only be a fraction of the medicament powder mass in an oral capsule. Thus, there is an increasing demand for better medicament compositions and filling methods for making small and exact inhalation doses with low relative standard deviation (RSD).

Volumetric filling is by far the most common method of producing doses of medication drugs. Normally in a first step a quantity of powder is introduced into a receptacle of specified volume by a mechanical device such as a piston or the receptacle may be filled by gravitation and/or suction force. Then in a second step the receptacle is moved to an unloading position, where e.g. the piston or an applied overpressure ejects the powder load out of the receptacle into a container such as a blister or capsule etc. A plurality of receptacles may be arranged in a dose-forming tool, which is adapted to a mechanism bringing a plurality of containers, e.g. blisters or capsules, in line with the corresponding receptacles so that doses of powder may be loaded into the containers. The dose-forming receptacle tool may be integrated into a filling machine such that the receptacles can be filled and emptied in a more or less continuous, cyclic fashion. Examples of prior art may be studied for instance in publications EP 0 319 131 B1, WO 95/21768, US 5,826,633, US 6,267,155 B1, US 6,581,650 B2, DE 202 09 156 U1, WO 03/026965 A1, WO 03/66436 A1 and WO 03/66437 A1.

The active substance in dry powder form, suitable for inhalation needs to be finely divided so that the majority by mass of particles in the powder is between 1 and 5 µm in aerodynamic diameter (AD). Powder particles larger than 5 µm tend not to deposit in the lung when inhaled but to stick in the mouth and upper airways, where they are medicinally wasted and may even cause adverse side effects. However, finely divided powders, suitable for inhalation, are rarely free flowing but tend to stick to all surfaces they come in contact with and the small particles tend to aggregate into lumps. This is due to van der Waal forces generally being stronger than the force of gravity acting on small particles having diameters of 10 µm or less. Therefore, metering and loading correct quantities of a dry, inhalable powder composition into a dose container, such as a blister for example, becomes more and more difficult the smaller the nominal dose mass gets. Because most active drugs are very potent, only a fraction of a milligram is needed in a dose in many cases. It is therefore necessary to dilute the drug using a suitable, physiologically inert excipient, e.g. lactose, before manufacturing of doses of the drug commences. Today, nominal inhalation doses of less than 1 mg and even less than 0.5 mg are not unusual. Such small doses are very difficult to meter and fill using prior art methods. See for instance the publication US 5,865,012 and WO 03/026965 A1. The problem of bad flowability in the powder is often addressed by selecting an excipient as diluent, which comprises bigger particles than the drug, i.e. aerodynamic particle diameters for the excipient larger than 10 µm. However, there is interaction between the active drug particles and the diluent, such that the size of the diluent particles plays a role, which affects not only flowability but also the small particle fraction of the delivered active drug to a user of a DPI. Thus, a balance between contradicting objectives must be struck. See for instance the publication WO 02/30389. A common practice in the pharmaceutical industry is to dilute the active substance further, in order to increase the nominal dose mass to a level, which the filling method of choice can handle. Typically, volumetric doses in prior art have masses in a range from 5 to 50 mg. This often means that the active substance is diluted by a thousand times or more. It is difficult to ascertain that the mix of active substance and diluent is homogenous and to ensure during dose filling that the amount of active substance in each and every one of the metered doses is correct. If the composition comprises big particles to improve flowability for example, care must be taken in handling the powder in order to avoid particle segregation, which easily happens during transportation and handling of the powder. Big particles tend to stay uppermost and small particles tend to fall to the bottom of a storage cavity, which of course results in inconsistent mixing ratios between the finely divided drug and the big particle excipient in the stored powder.

Turning to the drug formulation, there are a number of well-known techniques to obtain a appropriate primary particle size distribution to ensure correct lung deposition for a high percentage of the dose. Such techniques include jet-milling, spray-drying and super-critical crystallization. There are also a number of well-known techniques for modifying the forces between the particles and thereby obtaining a powder with appropriate adhesive forces. Such methods include modification of the shape and surface properties of the particles, e.g. porous particles and controlled forming of powder pellets, as well as addition of an inert carrier with a larger average particle size (so called ordered mixture). A simpler method of producing a finely divided powder is milling, which produces crystalline particles, while spray-drying etc produces amorphous particles. Novel drugs, both for local and systemic delivery, often include biological macromolecules, which put completely new demands on the formulation. In our publication WO 02/11803 (US 6,696,090) a method and a process is disclosed of preparing a so called electro-powder, suitable for forming doses by an electro-dynamic method. The disclosure stresses the importance of controlling the electrical properties of a medication powder and points to the problem of moisture in the powder and the need of low relative humidity in the atmosphere during dose forming.

In another aspect of prior art filling methods, the particle size of the selected diluent is chosen to be in a range from 10 to 200 µm, i.e. the excipient acts also as a carrier of the smaller, active particles. This makes the composed powder admixture much more flowable, which simplifies the filling of capsules or blisters considerably. A common volumetric filling method is to use a dose dispensing device or "dosator", as used in e.g. WO 03/066437 A1, or quite simply to let powder drop onto a carrier foil, which has been impressed with a multitude of cavities acting as metering cavities. A dosator may compact the powder to a predefined degree before pushing the dose into a receiving cup such as a capsule or blister for instance. But some powder at the open end of the dosator may drop off during transport to the receiving cup or powder may stick to other surfaces of the dosator such that particles falling off the dosator may create a dust cloud and stick to critical areas and surfaces, which are supposed to be clean. A surplus of powder is often arranged to fall into or fill the cavities, whereupon the surplus powder is wiped off from the carrier foil by e.g. a doctor blade, before a different foil, which is glued or fused onto the carrier foil, seals the cavities. The process has two inherent problems; the first is that the falling medicament powder emits a cloud of dust, whereupon dust particles then settle on other surfaces in the vicinity, including the sealing areas of the foils, the second problem is that the wiping action of a doctor blade is a very sensitive operation and may leave powder particles on the sealing areas, such that sealing is less than perfect for some of the blisters. Bad sealing may lead to premature deterioration of doses during storage, such that the effect of affected doses is not the intended one when inhaled by a user, potentially presenting serious problems to the user in need of treatment.

Yet another problem facing a user of the described prior art dose manufacturing methods is the problem of de-aggregating the powder composition when the dose is made available in a dry powder inhaler (DPI).

Because the first priority in manufacturing is to make doses of an almost free-flowing powder composition in order to achieve consistency between doses and a small variation between powder batches, the ability to de-aggregate the dose in a DPI does not get the same attention. The efficacy of the dose is therefore mediocre; the fine particle fraction of the delivered drug is often less than 25 %.

A more recent prior art method of forming a metered dose utilizes an electrostatic or electro-dynamic field deposition process or combinations thereof for depositing electrically charged particles of a medication powder onto a substrate member, such as an electrostatic chuck or a dosing member. A method of depositing microgram and milligram quantities of dry powders using electric field technology is disclosed in our US Patent No. 6,592,930 B2, which is hereby incorporated in this document in its entirety as a reference. The method is particularly suitable for forming small doses below 10 mg in mass. An example of a suitable dose of medication powder, formed onto a substrate member, is an electro-dose. The term electro-dose, presented in our Swedish Patent No. SE 0003082-5, which is hereby incorporated herein by reference, refers to a dose of pre-metered medicament powder intended for use in a dry powder inhaler. The electro-dose is formed from an electro-powder comprising an active powder substance or a dry powder medicament formulation with or without one or more excipients, the electro-dose being formed onto a substrate member, which is part of a dosing member. The so formed electro-dose presents appropriate properties in terms of occupied area, powder contour, particle size, mass, porosity, adhesion etc for easy de-aggregation and dispersal into air by the use of a suitable dry powder inhaler device.

However, there is still a need for improved medicament preparations and better adapted dose forming methods making the filling process an exact, reliable one for precise metering and forming of medicament doses of finely divided, dry powders for inhalation.

### SUMMARY

The present invention discloses a dry powder medicament preparation and methods of forming and loading metered, non-dusting, porous loads of joined particles of the preparation into dose containers intended for insertion into a dry powder inhaler. The doses are arranged for pro-longed delivery by inhalation, whereby a high delivered fine particle dose is emitted from the inhaler.

The disclosed preparation comprises at least one finely divided pharmacologically active ingredient having a mean particle diameter not less than 0.5 µm and not more than 6 µm and optionally at least one physiologically acceptable, dry, finely divided excipient. The preparation is adapted for a forming and loading process, which may be based on volumetric filling or on electro-dynamic deposition of powder particles, such that a metered dose of the preparation is characterized by containing one or more non-dusting, porous loads of joined particles in a macrostructure of predefined dimensions and having an intended mechanical strength.

In a further aspect of the invention, on-line or off-line inspection of the dose is made possible by applying one or more measurement systems e.g. optical vision systems, laser systems, near infrared systems, electric field systems and electric capacitance systems. Quality control is in this manner simplified.

The pharmacologically active ingredient presents at least 80 % and preferably at least 90 % by mass of particles in an aerodynamic diameter range from 1 to 10 µm and more preferably from 1 to 5 µm and most preferably from 1 to 3 µm, the latter range particularly desirable for systemically acting active ingredients.

Further, metered loads constituting a dose are advantageously formed and loaded into a selected type of dose container, preferably a high barrier container serving against moisture, in ambient conditions with normal room temperature and presenting less than 30 %, preferably less than 20 % and most preferably less than 10 % relative humidity.

Typically, the preparation generates pre-metered doses in a range from 0.1 to 50 mg and preferably from 0.5 to 25 mg.

Particular methods of forming and loading a metered dose use suitably adapted volumetric filling and metering and electro-dynamic dosing and metering of the disclosed dry powder preparation. The powder preparation and the porous loads thereof are particularly adjusted for prolonged delivery by a dry powder inhaler (DPI). Different prior art DPIs may be used e.g. types characterized by having a prolonged dose delivery, types incorporating an air-razor device and types having multiple dose containers in an elongated tape with a peelable sealing tape.

The present preparation is set forth by the independent claim 1 and the dependent claims 2 to 14, and methods of loading are set forth by the independent claims 15 and 25 and the dependent claims 16 to 24 and 26 to 33 respectively.

### BRIEF DESCRIPTION OF THE DRAWINGS

The invention, together with further objects and advantages thereof, may best be understood by referring to the following detailed description taken together with the accompanying drawings, in which:
FIG. 1 illustrates a flow diagram showing the steps of the claimed method of volumetric filling;
FIG. 2 illustrates a flow diagram showing the steps of the claimed method of electro-dynamic dosing;
FIG. 3 illustrates a stylized, principal drawing of a preferred embodiment of a filling tool and associated details;
FIG. 4 illustrates in principle a longitudinal section of an embodiment of the filling tool together with the air nozzles, the air supply lines and the relative positions of the tool, the powder in a storage chamber with powder release chutes and the containers to be filled;
FIG. 5 illustrates in a photograph an embodiment of two typical volumetric loads loaded onto a common dose bed of a dose container, according to the present invention;
FIG. 6 illustrates in a photograph a close-up of two typical volumetric doses having intact loads of joined particles and loaded onto a dose bed of a dose container, according to the present invention, and
FIG. 8 illustrates a stylized, principal drawing of an embodiment of an electro-dynamic dose forming method.

### DESCRIPTION OF THE INVENTION

The present invention discloses a dry powder medicament preparation and methods of forming and loading non-dusting, porous loads of joined particles constituting a pre-metered dose of the preparation into a dose container. The doses are intended for inhalation, for local lung deposition against respiratory disorders or for deep lung deposition and systemic action. The objective of the invention is to provide the preparation and the metered doses with the following qualities:
- a controlled cohesion between powder particles of the preparation
- a negligible inclination for dusting of the porous load
- ease of compacting the preparation for volumetric metering and filling even of relatively small doses
- ease of de-aggregating a coherent load of joined particles dispersed into air by inhalation
- preparation and metered dose suitable for a prolonged delivery and gradual aerosolization

In the context of this document all references to ratios, including ratios given as percentage numbers, are related to mass, if not explicitly said to be otherwise.

Surprisingly we have found by experimentation that a medicament preparation comprising a finely divided, dry powder, pharmacologically active ingredient optionally in a mixture with at least one physiologically acceptable, dry, finely divided excipient, may be advantageously used in a volumetric metering and filling process for producing consistent, metered doses of the medicament preparation. The pharmacologically active ingredient should present at least 80 % by mass and preferably at least 90 % by mass of particles in an aerodynamic diameter range from 1 to 10 µm and preferably from 1 to 5 µm and most preferably from 1 to 3 µm, the latter particularly desirable for ingredients intended for systemic absorption. For locally acting drugs, the preferred deposition of the drug in the lung depends on the location of the particular disorder, so depositions in the upper as well as the lower airways are of interest. For systemic delivery of the medication, a deep lung deposition of the drug is preferred and usually necessary for maximum efficiency. The expression "deep lung" should be understood to mean the peripheral lung and alveoli, where direct transport of active substance to the blood can take place.

The optional, finely divided excipient should have an average particle diameter smaller than 10 µm. Although flowability of the dry powder medicament preparation may be low, the prepared powder can be handled and made available in an intermediate reservoir for a filling operation. Particular methods of producing pre-metered doses of the preparation are disclosed in the following, whereby doses in a range from 0.1 to 50 mg and more preferably from 0.5 to 25 mg may be advantageously produced.

The chosen ratio between a pharmacologically active ingredient (API), which may be more or less potent, and an excipient is typically in a range from 10:1 to 1:200, depending on the potency of the active ingredient and with consideration to a preferred, targeted total dose mass, i.e. including the chosen excipient. For instance, in a case of a very potent ingredient such as tiotropium, where the dosage to a user would be typically 10 µg, a ratio of 1:99 would generate a total dose of 1 mg. In this example the chosen excipient is discussed from a diluting point of view, but the excipient may also contribute in other ways to a successful medicament preparation. From this point on, the term "excipient" is used to describe any chemical or biologic substance mixed in with a pure active agent for whatever purpose. The preparation may comprise several different physiologically acceptable, dry excipients, such as enhancers, carriers and diluents in order to give the preparation the desired properties. On the other hand, there are medicaments in existence, which require several tens of milligrams of pure pharmacologically active agent in a normal dose. In such cases it would not be necessary to add excipients to the active agent for the purpose of diluting the drug, although there may be other reasons for doing so.

The present invention can be advantageously applied to most types of drugs and it also discloses a possibility to include more than one pharmacologically active ingredients. Combined doses of two or more different medicaments are attracting interest in most therapeutic areas today, especially e.g. in treatment of asthma and chronic obstructive pulmonary disease (COPD) and pain control. However, dry medicament preparations will soon be available specifically adapted to state of the art dry powder inhalers (DPI), where the combination of a new preparation and a new DPI will typically bring the delivered fine particle dose up to more than 50 % by mass of the metered dose. Therefore, demand for systemic therapy based on DPIs is expected to rise dramatically in many medical areas in the near future.

Preferred dry powder inhalers for pre-metered doses are types offering a prolonged dose delivery, the advantages may be studied in the publication US 6,622,723 B1, types incorporating an air-razor device as disclosed in publication US-2003-0192538-A1 and types using blister-pack containers with a peelable seal foil as described in publication US 6,536,427 B2 the publications herewith included in their entirety in this document as references.

Typical, non-exclusive, illustrative examples not limiting the scope of the invention of suitable, pharmacologically active ingredients are selected from the group comprising vasopressin, a vasopressin analogue, desmopressin, glucagons-like peptides (GLP-1, GLP-2), corticotropin, gonadotropin, calcitonin, C-peptide of insulin, parathyroid hormone, human growth hormone, growth hormone, growth hormone releasing hormone, oxytocin, corticotropin releasing hormone, a somatostatin analogue, a gonadotropin agonist analogue, atrial natriuretic peptide, thyroxine releasing hormone, follicle stimulating hormone, prolactin, an interleukin, a growth factor, a polypeptide vaccine, an enzyme, an endorphin, a glycoprotein, a lipoprotein, a kinase, intra-cellular receptors, transcription factors, gene transcription activators/repressors, neurotransmitters, proteoglycans, a polypeptide involved in the blood coagulation cascade that exerts its pharmacological effect systemically, any other polypeptide having a molecular weight (Daltons) of up to 200 kDa proteins, polysaccharides, lipids, nucleic acids and combinations thereof or from the group consisting of leuprolide and albuterol, opiates nicotine, nicotine derivates, scopolamin, morphine, apomorphine analoges, sumatriptan, naratriptan, zolmitriptan, rizatriptan, almotriptan, eletriptan, frovatriptan, pharmaceutically active chemicals for respiratory disorders and salts thereof, such as formoterol, budesonide, ipratropium, fluticasone, tiotropium, salbutamol and mometasone.

The at least one physiologically acceptable excipient is normally selected from a group of substances comprising glucose, arabinose, lactose, lactose monohydrate, lactose unhydrous, saccharose, maltose, dextrane, sorbitol, mannitol, xylitol, natriumchloride, calciumcarbonate or mixtures thereof.

In prior art good flowability is in focus, because it is normally necessary to make a medicament composition suitable for gravitation filling, where the powder is poured by gravity into metering cavities or directly into blisters and capsules. In prior art, the amount of powder per dose is normally quite big, presuming high ratios between active drug and diluent. The present invention, on the other hand, focuses on attaining a very high, delivered fine particle dose from a chosen dry powder inhaler device. Consequently, the nominal metered dose mass is targeted from the viewpoint that the metered dose mass should be in a range where optimum performance from the inhaler can be expected. Modern, dry powder inhalers for pre-metered doses give their best performance for doses with masses at or below 10 mg approximately.

The selected nominal dose mass and the selected pharmacologically active ingredients and their respective dosages to be included in the nominal dose then sets the mass ratio between active ingredients and the optional physiologically acceptable excipients. Thus, a medicament preparation must be prepared according to these constraints and at the same time it should provide the necessary qualities for a successful adaptation to a preferred method of forming and loading metered doses into containers. A dry powder preparation must be possible to handle in a filling process, without too many problems, e.g. in the way of electrostatic charging of particles and associated risk of powder sticking and clogging, tendency of particles to agglomerate and form powder granules, varying bulk density in the composition making volume metering and filling unreliable etc. The smaller the average powder particle size gets, the more difficult it will be to handle the powder. The difficulty varies considerably between different powder compositions and depends on the actual powder and its properties. Large excipient particles, i.e. at least 15 - 20 µm in size, are often needed to give a homogenous dry powder mixture, consisting of a finely divided pharmacologically active drug and large excipient particles, a minimum of flowability. The small particles attach to the larger ones and the powder retains the properties of a powder composed of large particles.

Electrostatics is often a problem in handling of dry powders, especially finely divided powders. Fine particles are easily triboelectrically charged when transported, not only by contact with objects of the transportation system but also by flowing air. The problem is aggravated by the necessity of handling the powder in a dry atmosphere, at least below 30 % and preferably below 20 % relative humidity, in order not to affect the quality and properties of the powder. The powder particles may be electrically discharged by applying static elimination devices, e.g. from NRD LLC, Grand Island, New York. Such static elimination devices may be applied where needed in the different steps of a dosing process to keep static charging of the powder, the metering cavities and associated equipment to a minimum throughout the dosing procedure. Eliminating static charging keeps loss of particles due to particle-sticking and other interference from electrostatics in the dosing process to a minimum.

In a different aspect of the invention we have surprisingly found that it is possible to prepare the blend of pharmacologically active, respirable drugs and optional excipients into a medicament preparation capable of joining particles into a macro agglomeration structure not unlike a child's sandcastle. The preparation is particularly suitable for an adapted volumetric filling method, but the properties of the preparation may also be advantageous to an electric dosing method. In a particular embodiment a selected active pharmacologic ingredient is micronized by jet milling, which may optionally be repeated at least once. The resulting powder may be produced with a very narrow particle size distribution and may present a desired peak somewhere in the range 0.5 - 6 µm. Typically, as measured by a laser scattering method, e.g. a Malvern Mastersizer, the ratio between the 90 % diameter (D_{(v,0.9)}) and the 10 % diameter (D_{(v,0.1)}) is approximately 3.

Different APIs are more or less sensitive to moisture, small particles form easily aggregates in the presence of moisture, and aggregates may be quite difficult to de-aggregate. From a stability point of view, a solid powder preparation stored under dry conditions is normally the best choice also avoiding elevated temperatures. Generally, APIs in dry powder form suitable for inhalation are sensitive to moisture and protecting the metered medication dose from moisture all the way through the steps of filling, sealing, transporting and storing is an important aspect of the present invention.

A quantity of the medicament preparation may thus be formed into a coherent, but porous macro structure when the prepared powder is filled and lightly compacted into a specially formed metering cavity. Besides having a predetermined volume, the shape of the cavity is such that it forms the macro structure of the load, such that the resulting load contour geometry fits the shape and size of a chosen dose container. A conical, oblong cavity is preferred such as a truncated pyramid or ellipse, but a cylindrical cone is equally possible. The metered load is characterized in that it holds together, keeping the shape intact without disintegrating, when it is ejected from the metering cavity. Preferably, the load contour remains intact when the load is dropped onto a dose bed in a dose container after ejection. This eliminates the risk of particles in the load going astray in the transfer of the load from the metering cavity to a chosen container. Furthermore, no particle dust is then emitted when the load is dropped into the container. Contamination by stray dust particles of the sensitive sealing areas around the dose container is thereby eliminated. The need for frequent cleaning of a container carrier system, e.g. an elongated foil tape, the filling device and associated equipment is much reduced. Still, the compaction is not driven to a point where particles form agglomerates needing high levels of energy to de-agglomerate, but just enough so that the load structure is easily broken up, e.g. by agitating the container or adding energy to the load itself before the dose container is introduced into a DPI. But most preferably, de-aggregation of particle aggregates constituting the load macro structure, takes place in a selected, adapted DPI when the dose is delivered to an inhaling user. In that case the delivered fine particle dose of the active drugs in the metered dose is maintained at more than 30 % and preferably more than 40 % and most preferably more than 50 % of the metered active drug dose.

Surprisingly, we have found by experimentation that the delivered fine particle dose, FPD, of the disclosed preparation is strongly dependent on the timing of the delivery within the inhalation cycle. Ideally, delivery should not begin until the suction provided by the user has exceeded approximately 2 kPa. Concentrating the suction energy to the precise areas where the loads of the preparation are located, provides a high, local airflow speed, which is adequate for complete aerosolization and de-aggregation of the loads. It is particularly advantageous to use an adapted DPI releasing the loads of the dose in a prolonged interval, i.e. the dose is arranged to be released gradually and not all loads of the preparation at once. The dose is preferably adapted for prolonged delivery within a time frame of not less than 0.1 second and not more than 5 seconds, preferably in a range 0.2 - 2 seconds. An example of a suitable inhaler is disclosed in our U.S. Patent No. 6,422,236 B1 and principles of inhaler design are disclosed in our U.S. Patent No. 6,571,793 B1.

An electro-dynamic method using electric field technology for dosing electrically charged particles of a medication powder directly into the container may be an alternative to volumetric filling methods. In such case the preparation needs to meet electric criteria besides the chemical, biological and physical criteria discussed in the foregoing. A preferred electro-dynamic method uses at least one particle transfer electrode arranged for forming an electric iris diaphragm and shutter with an electric field associated for the transfer of the powder particles from a powder reservoir. Particles are picked up from the reservoir by suitable means, e.g. a brush, and given an electric charge, e.g. by triboelectricity, and then introduced into an electric field, which transports the particles to the dose bed of a chosen container where they are deposited. The container is arranged to accept a metered powder dose, directly deposited by the electro-dynamic method, which controls the deposition of particles in the dose forming or loading process. By controlling the electric charge of the particles, the strength of the electric field, the particle flow and the spatial deposition of the particles it is possible to control the mass density, i.e. porosity, of the dose such that the macro structure of the dose body gets the intended physical contour and the right mechanical strength.

A preferred embodiment of the dose container is a high barrier container i.e. a container presenting a high barrier seal against moisture. A dose bed is normally an integral part of the high barrier container. The high barrier container should preferably be made out of a type of aluminum foil approved to be in direct contact with pharmaceutical products. Aluminum foils that work properly in these aspects generally consist of technical polymers laminated with aluminum foil to give the foil the correct mechanical properties to avoid cracking of the aluminum during forming. Sealing of the formed containers is normally done by using a thinner cover foil of pure aluminum or laminated aluminum and polymer. The container and cover foils are then sealed together using at least one of several possible methods, for instance:
- using a heat sealing lacquer, through pressure and heat;
- using heat and pressure to fuse the materials together;
- ultrasonic welding of the materials in contact.
Any contamination by particles of the sealing surfaces jeopardizes the high barrier seal quality and must therefore be avoided. A metered load of the preparation, which holds together in a macro structure according to the present invention helps to accomplish this objective.

In a further aspect of the invention the loads making up the dose loaded into a container represents a measuring object. The contour of the loads have the shape and size of the corresponding metering cavity or the given shape and size from the electro-dynamic deposition process. This makes it possible to measure the metered dose mass in the container before sealing by applying e.g. optical systems like lasers, vision systems or NIR-systems, but profiling systems operating by ultrasound, electric field or capacitance principles are equally possible. By measuring the metered mass of the doses, either on-line or off-line, it will be possible to verify the relative standard deviation (RSD) between doses and to check that the average dose is close to the target and that the number of doses, which are outside set limits in a batch is tolerable. A measurement system also makes it possible to reject doses, which are outside specifications for any reason.

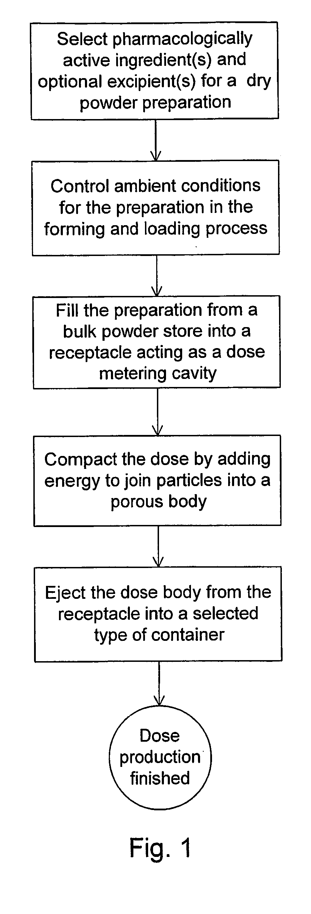

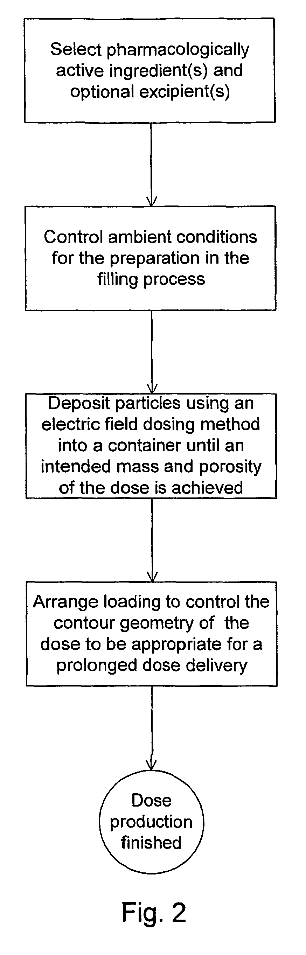

A flow diagram showing the steps of the claimed method of volumetric filling is illustrated in Figure 1 and a flow diagram of the claimed method of electro-dynamic dosing is illustrated in Figure 2.

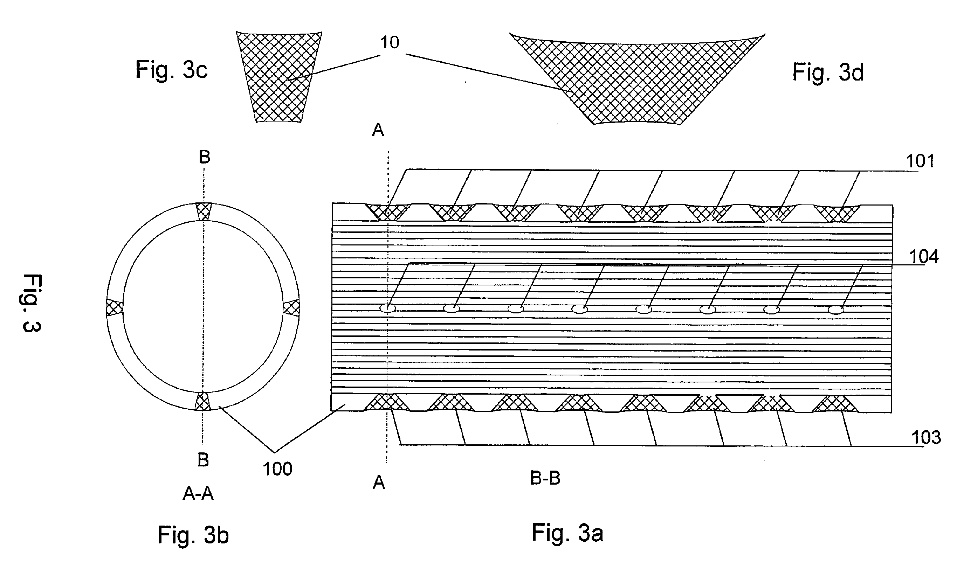

Figure 3 illustrates in 3(a) a cross-section A-A and in 3(b) a cross section B-B of an example of a filling tool for volumetric metering of loads. Enlarged cross-sections A 3(d) and B 3(c) of a receptacle 10 are also shown.

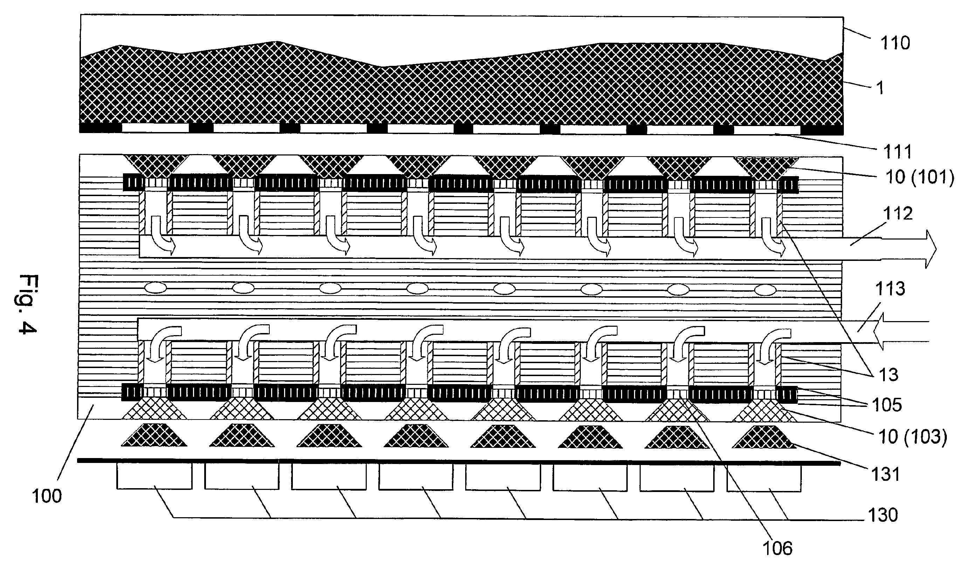

Figure 4 illustrates a stylized, principal drawing of a preferred embodiment of a filling tool 100 in a longitudinal cross-section together with a typical storage chamber 110 positioned above and in close proximity to the filling tool, a simple chute arrangement 111 for releasing powder 1 from the storage chamber to each individual receptacle 10, representing a metering cavity, in set 101. A multitude of containers 130 are positioned beneath receptacles 10 of set 103 and just ejected loads 131 are in the air on their way to their respective containers. Also shown are flexible seals 105, woven filters 106, air nozzles 13 and connecting air lines 112 and 113 for suction 114 during filling and air pressure 115 during unloading, respectively. Separate air lines are shown in the embodiment to simplify the reader's understanding of the principle, but in practice the same air line may be used alternately for suction and pressure during a complete sequence of filling and unloading.

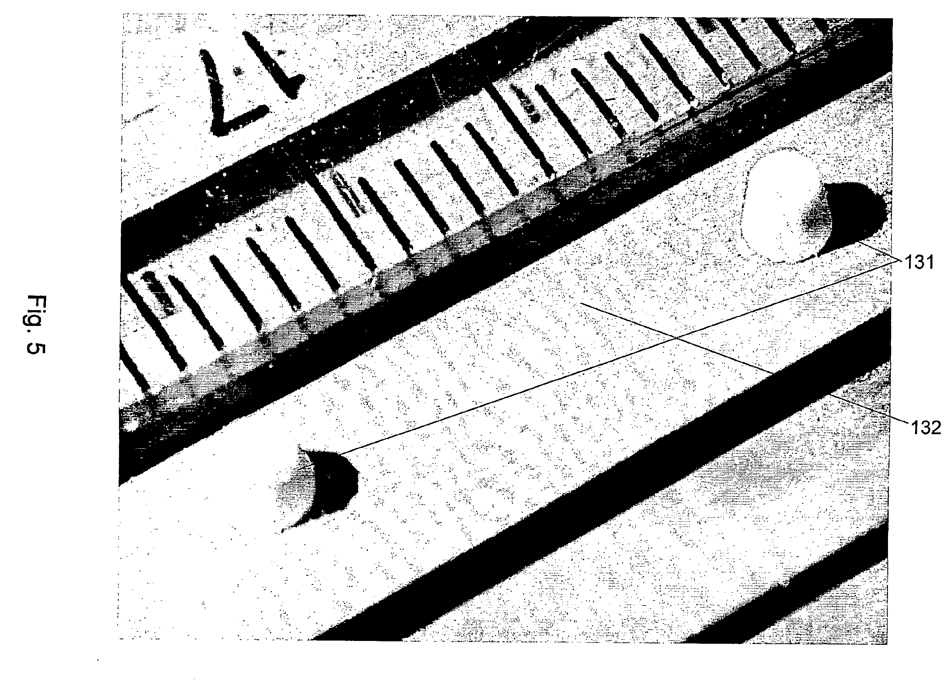

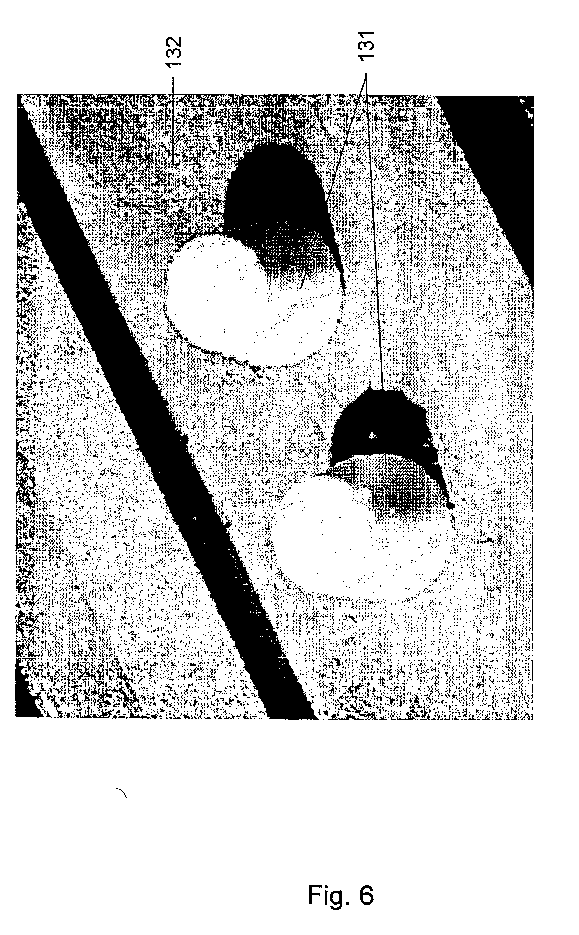

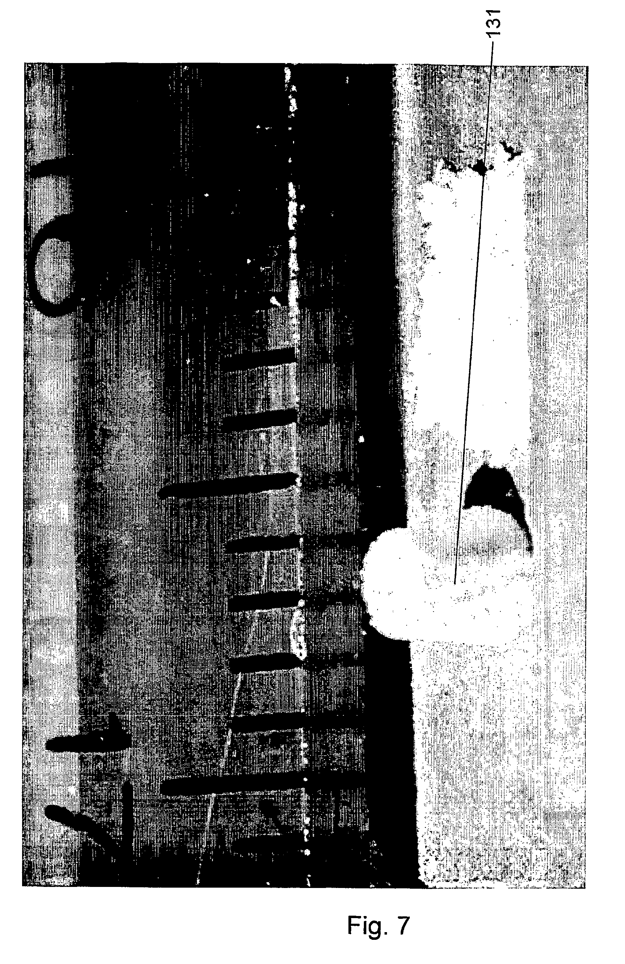

Two volumetrically metered loads 131, each with a mass of 4.5 mg, are illustrated in Figure 5. The loads are loaded onto a common dose bed 132, part of a container, the loads still having the geometric body structure intact A ruler with divisions per millimetre is included in the illustration to give an idea of the physical size of container and loads in the example. Close-ups of two metered loads 131, similar to Figure 5, loaded onto a common dose bed 132 having a body shape given by the filling receptacle 10 are illustrated in Figure 6. A metered load 131 and a pile of powder of the same preparation, although not compacted, representing the same mass as the metered dose are illustrated in Figure 7, which includes the aforementioned ruler to give some information regarding dimensions.

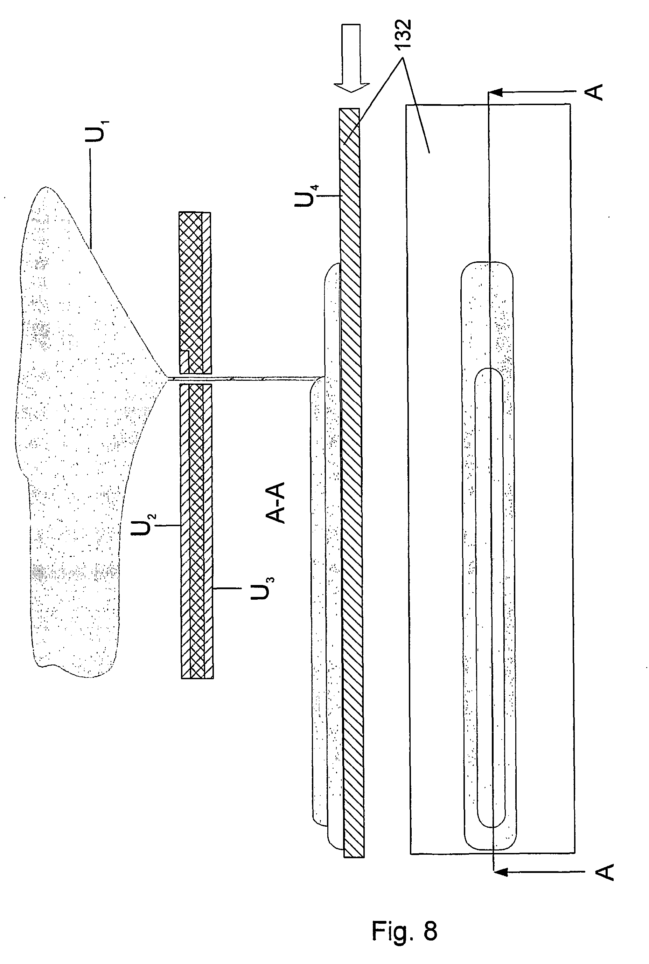

An electro-dynamic method of dosing particles onto a dose bed 132 are illustrated in Figure 8. Different voltages U1 - U4 are used to build up electric fields, which control particle transfer onto the dose bed, which may move relative a powder store, thereby making it possible to build up a chosen dose contour, a dose body, optionally in more than one layer.

In a preferred embodiment of the volumetric filling method, an elongated filling tool comprises at least one, but preferably more, precise receptacle functioning as a metering cavity or cup. Each receptacle has a first end and a second end. The smaller, second end is lined up with and connected to a nozzle, which in turn is connected to a supply of vacuum and compressed air through at least one fast acting on-off valve. For the sake of simplicity, the valve(s) may be common to all nozzles. Filling the receptacle(s) is accomplished by making powder available to the receptacle(s), e.g. through a chute arrangement from a storage chamber, such as a trough or a hopper. Normally powder is fed by gravitation, optionally aided by addition of energy, e.g. by vibrating the trough. When the tool containing the receptacle(s) has brought at least one receptacle in position to be filled, suction is applied from a vacuum source to the respective air nozzle, which in turn sucks powder falling from the chute into the receptacle, compacting the powder load to a degree in the receptacle. The suction force is set such that the powder load is lightly compacted into a coherent but porous dose body filling the receptacle completely. A special woven filter stops powder from entering the nozzle. After completing filling of some or all receptacles of the filling tool, the tool is cleaned from surplus powder and moved to a downward pointing position for unloading the dose body out of at least one receptacle into a selected container. When a valve opens, a pulse of compressed air is led through at least one nozzle and filter to the at least one receptacle, where the air exerts a force on the powder body in the receptacle. The dose is thereby ejected from the receptacle and drops into the selected container, provided it is in correct position to receive the dose. If the tool contains a plurality of receptacles it is advantageous to control the channeling of compressed air to the receptacles one by one in turn, but tight control of air pressure may also eliminate the risk of momentary dropping air pressure during unloading, which otherwise may result in uneven ejection of doses.

## Claims

1. A preparation of a dry powder medicament comprising at least one pharmacologically active ingredient, the preparation intended for aerosolization by a dry powder inhaler, **characterized in that**
the at least one active ingredient is presented having a mean particle diameter not less than 0.5 µm and not more than 6 µm;
the preparation is adapted for use in a forming process where the preparation is metered and by gentle force made into a non-dusting, porous load (131) of individual, non-agglomerated but coherent particles, and
one or more porous loads (131) of the preparation, loaded into a dose container (130), constitute a metered medicament dose adapted for a prolonged dose delivery using the dry powder inhaler.

2. The preparation according to claim 1, **characterized in that**
at least one biologically acceptable excipient is included in the preparation.

3. The preparation according to claim 1, **characterized in that**
the one or more porous loads (131), constituting the metered dose, are adjusted to a gradual de-aggregation and dispersal into an inhalation airflow resulting from an act of inhalation performed by using the dry powder inhaler.

4. The preparation according to claim 1, **characterized in that**
the at least one active ingredient presents at least 80 % and preferably at least 90 % by mass of particles in an aerodynamic diameter range from 0.1 to 10 µm and more preferably from 0.1 to 5 µm and most preferably from 0.1 to 3 µm, the latter range particularly desirable for systemically acting active ingredients.

5. The preparation according to claim 1, **characterized in that**
a targeted nominal active pharmacologic ingredient generates a pre-metered dose of the preparation in a range from 0.1 to 50 mg and preferably in a range from 0.5 to 25 mg.

6. The preparation according to claim 1, **characterized in that**
the dose container (130) has high barrier seal properties making it impervious to moisture and other foreign matter.

7. The preparation according to claim 1, **characterized in that**
a dose of the preparation is metered and loaded onto the dose bed (132) being part of a high barrier container (130) in ambient conditions with relative humidity less than 30 %, preferably less than 20 % and most preferably less than 10 %.

8. The preparation according to claim 1, **characterized in that**
the forming process is chosen to be an electric field dosing process (ELFID) employing a method of using electric fields and electrically charged medicament particles of the medicament preparation to form a pre-metered dose (131) directly onto a dose bed (132) being part of a selected type of dose container (130).

9. The preparation according to claim 1, **characterized in that**
the forming process is chosen to be a volumetric method using gravitation and optionally electric, mechanical or pneumatic energy for metering and filling loads (131) of the medicament preparation, thereby forming a pre-metered dose into a selected type of dose container (130).

10. The preparation according to claim 1, **characterized in that**
the at least one active pharmacologic ingredient is selected from a group of substances comprising vasopressin, a vasopressin analogue, desmopressin, glucagons-like peptides, corticotropin, gonadotropin, calcitonin, C-peptide of insulin, parathyroid hormone, human growth hormone, growth hormone, growth hormone releasing hormone, oxytocin, corticotropin releasing hormone, a somatostatin analogue, a gonadotropin agonist analogue, atrial natriuretic peptide, thyroxine releasing hormone, follicle stimulating hormone, prolactin, an interleukin, a growth factor, a polypeptide vaccine, an enzyme, an endorphin, a glycoprotein, a lipoprotein, a kinase, intra-cellular receptors, transcription factors, gene transcription activators/repressors, neurotransmitters, proteoglycans, a polypeptide involved in the blood coagulation cascade that exerts its pharmacological effect systemically, any other polypeptide having a molecular weight (Daltons) of up to 200 kDa proteins, polysaccharides, lipids, nucleic acids and combinations thereof or from the group consisting of leuprolide and albuterol, opiates nicotine, nicotine derivates, scopolamin, morphine, apomorphine analoges, sumatriptan, rizatriptan, almotriptan, eletriptan, frovatriptan, active chemicals for respiratory disorders and salts thereof, such as formoterol, budesonide, ipratropium, fluticasone, tiotropium, salbutamol and mometasone.

11. The preparation according to claim 1, **characterized in that**
the at least one physiologically acceptable, dry, finely divided excipient is selected from a group of substances comprising glucose, arabinose, lactose, lactose monohydrate, lactose unhydrous, saccharose, maltose, dextrane, sorbitol, mannitol, xylitol, natriumchloride, calciumcarbonate or mixtures thereof.

12. The preparation according to claim 1, **characterized in that**
at least one physiologically acceptable, solid excipient having a particle mass median aerodynamic diameter bigger than 20 µm is optionally added to the mixing step, the excipient selected from a group of substances comprising glucose, arabinose, lactose, lactose monohydrate, lactose unhydrous, saccharose, maltose, dextrane, sorbitol, mannitol, xylitol, natriumchloride, calciumcarbonate or mixtures thereof.

13. The preparation according to claim 1, **characterized in that**
a dry powder inhaler incorporating an Air-razor device is selected for emitting a selected, metered dose.

14. The preparation according to claim 1, **characterized in that**
a dry powder inhaler device is selected for emitting a selected, metered dose, the device being adapted to dose containers (130) in form of blisters sealed by peelable foil, which is to be peeled off prior to administering the dose to the user inhaling through the inhaler device.

15. A method of forming and loading a volumetrically metered dose (131) of a dry powder preparation into a selected type of dose container (130), the dose intended for a selected dry powder inhaler, **characterized by** the steps of
selecting the preparation to comprise at least one pharmacologically active, dry, finely divided ingredient having a mean particle diameter not less than 0.5 µm and not more than 6 µm;
controlling the ambient conditions for the preparation during forming and loading a metered dose, such that the relative humidity is kept below 30 %, preferably below 20 % and most preferably below 10 %;
filling the preparation from a bulk powder store (110) into at least one powder receptacle (10) acting as a metering cavity;
applying light force onto the load by adding energy in order to bring the powder particles together into a porous load (131) of non-agglomerated but coherent particles in the metering cavity, and
ejecting the load (131), the contour of which presents a defined geometry, into the selected type of container (130), such that the dose body is prevented from disintegrating into a pile.

16. The method of forming according to claim 15, **characterized by** the further step of
requesting that the at least one active ingredient presents at least 80 % and preferably at least 90 % by mass of particles in an aerodynamic diameter range from 0.1 to 10 µm and more preferably from 0.1 to 5 µm and most preferably from 0.1 to 3 µm, the latter range particularly desirable for systemically acting active ingredients.

17. The method according to claim 15, **characterized by** the further step of
lightly forcing powder particles into the dose metering cavity (10) making a porous dose load (131) of coherent particles, such that particles from the load (131) cannot spread outside the container (130) when the load (131) is ejected, thereby preventing particles from contaminating sealing surfaces of the container before a sealing step.

18. The method according to claim 15 **characterized by** the further step of
producing a metered dose of the preparation with mass in a range 0.1 to 50 mg and preferably in a range from 0.5 to 25 mg.

19. The method according to claim 15 **characterized by** the further step of
selecting a dry powder inhaler incorporating an Air-razor device for administering a selected, metered dose to a user, whereby a delivered fine particle dose constitutes by mass at least 30 % and preferably more than 40 % and most preferably 50 % of the total, pharmacologically active ingredient.

20. The method according to claim 15 **characterized by** the further step of
selecting a dry powder inhaler device providing a prolonged delivery of a selected, metered dose to a user.

21. The method according to claim 15 **characterized by** the further step of
selecting a dry powder inhaler device adapted to dose containers in form of blisters sealed by peelable foil, which is to be peeled off prior to administering a selected, metered dose to a user inhaling through the inhaler device.

22. The method according to claim 15 **characterized by** the further step of
using a high barrier sealing of the dose container (130) that will keep the dose unaffected by normal changes in ambient conditions for a specified time in storage before use and a specified time in use.

23. The method according to claim 15 **characterized by** the further step of
using suction power (114) and pressurized air (115) as the added force energy when making the load (131).

24. The method according to claim 15, **characterized by** the further step of
arranging sources of electric charges, preferably ion sources, at a working distance to the dose receptacle (10) and optionally at a working distance to the powder (1) in the bulk powder store (110) in order to accomplish that electrostatic charges on a filling tool (100) and associated equipment and powder particles in the store (110) become electrically neutralized such that the filling process is not adversely affected.

25. An electro-dynamic loading of a metered dose (131) of a dry powder medicament preparation into a selected type of container (130), the dose intended for a selected dry powder inhaler, **characterized by**
selecting the preparation to be loaded to comprise at least one pharmacologically active, dry, finely divided ingredient having a mean particle diameter not less than 0.5 µm and not more than 6 µm and optionally at least one physiologically acceptable, dry, finely divided excipient;
controlling the ambient conditions for the preparation during loading of the metered dose, such that the relative humidity is kept below 30 %, preferably below 20 % and most preferably below 10 %;
depositing particles of the preparation onto a dose bed (132), being part of the selected container (130), using an electric field dosing method until an intended pre-metered mass and correct porosity of the dose (131) is achieved;
arranging the loading such that the contour of the dose (131) presents a pre-defined geometry appropriate for a prolonged dose delivery by using the selected dry powder inhaler.

26. The loading according to claim 25, **characterized by**
the at least one active ingredient presents at least 80 % and preferably at least 90 % by mass of particles in an aerodynamic diameter range from 0.1 to 10 µm and more preferably from 0.1 to 5 µm and most preferably from 0.1 to 3 µm, the latter range particularly desirable for systemically acting active ingredients.

27. The loading according to claim 25, **characterized by**
selecting the dose container (130) to be a high barrier seal container, and
sealing the dose container (130) with a high barrier seal foil, thus enclosing the dose (131) in a tightly sealed package in order to keep the dose (13!) unaffected by normal changes in ambient conditions for a specified time.

28. The loading according to claim 25, **characterized by**
controlling the deposition of powder particles such that particles involved in the electric field dosing process cannot spread outside the high barrier container (130), thereby preventing particles from contaminating sealing surfaces of the container before a sealing step.

29. The loading according to claim 25, **characterized by**
producing a metered dose (131) of the preparation with mass in a range 0.1 to 50 mg and preferably in a range from 0.5 to 25 mg.

30. The loading according to claim 25, **characterized by**
selecting a dry powder inhaler incorporating an Air-razor device for delivering a selected, pre-metered dose, whereby an emitted fine particle dose constitutes by mass at least 30 % and preferably more than 40 % and most preferably more than 50 % of the total, pharmacologically active ingredient of the metered dose.

31. The loading according to claim 25, **characterized by**
selecting a dry powder inhaler device adapted to dose containers (130) in form of blisters sealed by peelable foil, which is to be peeled off prior to administering a selected, metered dose (131) to a user inhaling through the inhaler device.

32. The loading according to claim 25, **characterized by**
arranging sources of electric charges, preferably ion sources, at a working distance to the dose bed (132) and optionally at a working distance to the powder (1) in the bulk powder store (110) in order to accomplish that electrostatic charges on equipment associated with the dosing process and powder particles in the store become electrically neutralized such that the dosing process is not adversely affected.

## Patentansprüche

1. Zubereitung eines Trockenpulvermedikaments, die mindestens einen pharmakologisch aktiven Inhaltsstoff enthält, wobei die Zubereitung zur Versprühung durch einen Pulverinhalator vorgesehen ist, **dadurch gekennzeichnet, dass**
- der mindestens eine aktive Inhaltsstoff einen mittleren Partikeldurchmesser von nicht weniger als 0,5 µm und nicht mehr als 6 µm aufweist;
- die Zubereitung für die Verwendung in einem Umformvorgang geeignet ist, wobei die Zubereitung dosiert und durch sanfte Kraft in eine nicht staubende, poröse Ladung (131) von einzelnen, nicht agglomerierten, aber kohärenten Partikeln umgewandelt wird, und
- eine oder mehrere poröse Ladungen (131) der Zubereitung, die in einen Portionsbehälter (130) geladen wurden, eine dosierte Medikamentdosis bilden, die für eine anhaltende Dosisabgabe unter Verwendung eines Pulverinhalators geeignet ist.

2. Zubereitung nach Anspruch 1, **dadurch gekennzeichnet, dass** mindestens ein biologisch annehmbarer Hilfsstoff in der Zubereitung enthalten ist.

3. Zubereitung nach Anspruch 1, **dadurch gekennzeichnet, dass** eine oder mehrere poröse Ladungen (131), die die dosierte Dosis bilden, auf eine stufenweise Deaggregation und Verteilung in einen Inhalationsluftstrom abgestimmt sind, der durch den Vorgang der Inhalation resultiert, die unter Verwendung des Pulverinhalators ausgeführt wird.

4. Zubereitung nach Anspruch 1, **dadurch gekennzeichnet, dass** der mindestens eine aktive Inhaltsstoff zu mindestens 80 Gew.-% und bevorzugt zu mindestens 90 Gew.-% als Partikel in einem aerodynamischen Durchmesserbereich von 0,1 bis 10 µm und bevorzugter von 0,1 bis 5 µm, und am meisten bevorzugt von 0,1 bis 3 µm vorliegt, wobei der letztgenannte Bereich insbesondere für systemisch wirkende aktive Inhaltsstoffe wünschenswert ist.

5. Zubereitung nach Anspruch 1, **dadurch gekennzeichnet, dass** ein zielgerichteter nominell aktiver pharmakologischer Inhaltsstoff eine vordosierte Dosis der Zubereitung in einem Bereich von 0,1 bis 50 mg und bevorzugt in einem Bereich von 0,5 bis 25 mg erzeugt.

6. Zubereitung nach Anspruch 1, **dadurch gekennzeichnet, dass** der Portionsbehälter (130) hohe Barriereabdichtungseigenschaften aufweist, die ihn undurchlässig gegenüber Feuchtigkeit und anderen fremden Materialien machen.

7. Zubereitung nach Anspruch 1, **dadurch gekennzeichnet, dass** eine Dosis der Zubereitung auf ein Dosierbett (132), das Teil eines Behälters mit starken Sperreigenschaften (130) ist, bei Umgebungsbedingungen mit einer relativen Feuchtigkeit von weniger als 30%, bevorzugt weniger als 20%, und am meisten bevorzugt weniger als 10%, dosiert und geladen wird.

8. Zubereitung nach Anspruch 1, **dadurch gekennzeichnet, dass** der Umformvorgang ausgewählt ist aus einem elektrischen Felddosierverfahren (ELFID), wobei ein Verfahren unter Verwendung von elektrischen Feldern und elektrisch geladenen Medikamentpartikeln der Medikamentzubereitung angewendet wird, um eine vordosierte Dosis (131) direkt auf einem Dosierbett (132) zu bilden, das Teil eines ausgewählten Typs von Portionsbehälter (130) ist.

9. Zubereitung nach Anspruch 1, **dadurch gekennzeichnet, dass** der Umformvorgang ein volumetrisches Verfahren unter Verwendung von Gravitation und optional elektrischer, mechanischer oder pneumatischer Energie für die Dosierung und das Füllen von Ladungen (131) der Medikamentzubereitung ist, wobei eine vordosierte Dosis in einem ausgewählten Typ von Portionsbehälter (130) gebildet wird.

10. Zubereitung nach Anspruch 1, **dadurch gekennzeichnet, dass** der mindestens eine aktive pharmakologische Inhaltsstoff ausgewählt ist aus einer Gruppe von Substanzen, wie Vasopressin, einen Vasopressin-Analogon, Desmopressin, Glukagon-ähnlichen Peptiden, Corticotropin, Gonadotropin, Calcitonin, C-Peptid von Insulin, Nebenschilddrüsehormon, menschlichem Wachstumshormon, Wachstumshormon, Wachstumshormon freisetzendem Hormon, Oxytocin, Corticotropin freisetzendem Hormon, einem Somatostatinanalogon, einem Gonadotropinagonistanalogon, atrial natriuretischem Peptid, Thyroxin freisetzendem Hormon, Follikel stimulierendem Hormon, Prolactin, einem Interleukin, einem Wachstumsfaktor, einem Polypeptidimpfstoff, einem Enzym, einem Endorphin, einem Glykoprotein, einem Lipoprotein, einer Kinase, intrazellulären Rezeptoren, Transkriptionsfaktoren, Gentransskriptionsaktivatoren/repressoren, Neurotransmittern, Proteoglykanen, einem Polypeptid, das an der Blutgerinnungskaskade beteiligt ist, das seinen pharmakologischen Effekt systemisch ausübt, jedem anderen Polypeptid, das ein Molekulargewicht (Dalton) von bis zu 200 kDa der Proteine hat, Polysacchariden, Lipiden, Nukleinsäuren und Kombinationen davon oder aus der Gruppe, bestehend aus Leuprolid und Albuterol, Opiaten, Nikotin, Nikotinderivaten, Scopolamin, Morphin, Apomorphinanalogen, Sumatriptan, Rizatriptan, Almotriptan, Eletriptan, Frovatriptan, aktiven Chemikalien für Lungenfunktionsstörungen und Salzen davon, wie Formoterol, Budesonid, Ipratropium, Fluticason, Tiotropium, Salbutamol und Mometason.

11. Zubereitung nach Anspruch 1, **dadurch gekennzeichnet, dass** das mindestens ein physiologisch annehmbarer, trockener, fein zerteilter Hilfsstoff ausgewählt aus der Gruppe von Substanzen umfassend Glukose, Arabinose, Laktose, Laktosemonohydrat, wasserfreie Laktose, Saccharose, Maltose, Dextran, Sorbitol, Mannitol, Xylitol, Natriumchlorid, Calciumcarbonat und Mischungen davon.

12. Zubereitung nach Anspruch 1, **dadurch gekennzeichnet, dass** mindestens ein physiologisch annehmbarer, fester Hilfsstoff, dessen Partikel einen massenmittleren aerodynamischen Durchmesser größer als 20 µm haben, optional zu dem Mischschritt zugegeben wird, wobei der Hilfsstoff ausgewählt ist aus der Gruppe von Substanzen, umfassend Glukose, Arabinose, Laktose, Laktosemonohydrat, wasserfreie Laktose, Saccharose, Maltose, Dextran, Sorbitol, Mannitol, Xylitol, Natriumchlorid, Calciumkarbonat und Mischungen davon.

13. Zubereitung nach Anspruch 1, **dadurch gekennzeichnet, dass** der Pulverinhalator, der eine Luftzerteilvorrichtung beinhaltet, dazu vorgesehen ist, eine ausgewählte, dosierte Dosis zu emittieren.

14. Zubereitung nach Anspruch 1, **dadurch gekennzeichnet, dass** die Pulverinhalationsvorrichtung dazu ausgewählt ist, eine ausgewählte dosierte Dosis zu emittieren, wobei die Vorrichtung an Portionsbehälter (130) in Form von mit abziehbarer Folie versiegelten Blistern angepasst ist, wobei die Folie vor der Verabreichung der Dosis an den Anwender, der durch die Inhalationsvorrichtung inhaliert, abgezogen wird.

15. Verfahren zur Bildung und Beladung einer volumetrisch dosierten Dosis (131) einer Trockenpulverzubereitung in einen ausgewählten Typ von Portionsbehälter (130), wobei die Dosis für einen ausgewählten Pulverinhalator bestimmt ist, **gekennzeichnet durch** die Schritte
- Auswählen der Zubereitung, so dass sie mindestens einen pharmakologisch aktiven, trockenen, fein zerteilten Inhaltsstoff mit einem mittleren Partikeldurchmesser von nicht weniger als 0, 5 µm und nicht mehr als 6 µm, enthält;
- Kontrollieren der Umgebungsbedingungen für die Herstellung während des Bildens und Beladens einer dosierten Dosis, so dass die relative Feuchtigkeit unter 30%, bevorzugt unter 20%, und am meisten bevorzugt unter 10% gehalten wird;
- Abfüllen der Zubereitung aus einem Bulkpulverspeicher (110) in mindestens einen Pulverbehälter (10), der als Dosierraum dient;
- Anwenden einer leichten Kraft auf die Ladung **durch** Zuführung von Energie, um die Pulverpartikel zusammen in eine poröse Ladung (131) von nicht agglomerierten, aber kohärenten Partikeln in dem Dosierraum zu bringen, und
- Ausstoßen der Ladung (131), deren Umriss eine definierte Geometrie aufweist, in einen ausgewählten Typ von Behälter (130), so dass der Portionskörper vor dem Zerfall in einen Haufen bewahrt ist.

16. Verfahren zur Bildung nach Anspruch 15, **gekennzeichnet durch** den weiteren Schritt der
- Anforderung, dass bei dem mindestens einen aktiven Inhaltsstoff mindestens 80 Gew.-% und bevorzugt mindestens 90 Gew.-% der Partikel in einem aerodynamischen Durchmesserbereich von 0,1 bis 10 µm und bevorzugter von 0,1 bis 5 µm und am meisten bevorzugt von 0,1 bis 3 µm vorliegen, wobei der letztgenannte Bereich insbesondere für systemisch wirkende aktive Inhaltsstoffe wünschenswert ist.

17. Verfahren nach Anspruch 15, **gekennzeichnet durch** den weiteren Schritt des
- leichten Treibens der Pulverpartikel in den Portionsdosierraum (10), wodurch eine poröse Portionsladung (131) von kohärenten Partikeln gebildet wird, so dass sich die Partikel aus der Ladung (131) nicht außerhalb des Behälter (130) verteilen können, wenn die Ladung (131) ausgestoßen wird, wobei die Partikel an der Kontaminierung der Siegeloberflächen des Behälters vor dem Versiegelungsschritt gehindert werden.

18. Verfahren nach Anspruch 15, **gekennzeichnet durch** den weiteren Schritt der
- Herstellung einer dosierten Dosis der Zubereitung mit einer Masse in einem Bereich von 0,1 bis 50 mg und bevorzugt in einem Bereich von 0,5 bis 25 mg.

19. Verfahren nach Anspruch 15, **gekennzeichnet durch** den weiteren Schritt des
- Auswählens eines Pulverinhalators, der eine Luftzerteilvorrichtung enthält, zur Verabreichung einer ausgewählten, dosierten Dosis an einen Anwender, wobei eine abgegebene feine Partikeldosis aus mindestens 30% und bevorzugt mehr als 40% und am meisten bevorzugt 50% des gesamten pharmakologisch aktiven Inhaltsstoffes besteht.

20. Verfahren nach Anspruch 15, **gekennzeichnet durch** den weiteren Schritt des
- Auswählens einer Pulverinhalationsvorrichtung, die eine anhaltende Abgabe einer ausgewählten, dosierten Dosis an einen Anwender bereitstellt.

21. Verfahren nach Anspruch 15, **gekennzeichnet durch** den weiteren Schritt des
- Auswählens einer Pulverinhalationsvorrichtung, die an Portionsbehälter in Form von Blistern, die mit einer abziehbaren Folie versiegelt sind, angepasst ist, wobei die Folie vor der Verabreichung einer ausgewählten, dosierten Dosis an einen Anwender, der **durch** eine Inhalationsvorrichtung inhaliert, abgezogen wird.

22. Verfahren nach Anspruch 15, **gekennzeichnet durch** den weiteren Schritt der
- Verwendung einer Hochleistungssperrversiegelung des Portionsbehälters (130), die die Dosis für eine spezifische Zeit der Lagerung vor der Verwendung und eine spezifische Zeit während der Verwendung unbeeinflusst durch normale Veränderungen in den Umgebungsbedingungen erhält.

23. Verfahren nach Anspruch 15, **gekennzeichnet durch** den weiteren Schritt der
- Verwendung von Zugkraft (114) und unter Druck gesetzter Luft (115) als zugeführte treibende Energie, wenn die Ladung (131) gebildet wird.

24. Verfahren nach Anspruch 15, **gekennzeichnet durch** den weiteren Schritt der
- Anordnung von Quellen für elektrische Ladung, bevorzugt Ionenquellen, in einem Arbeitsabstand zu dem Portionsbehälter (10) und optional in einem Arbeitsabstand zu dem Pulver (1) in dem Bulkpulverspeicher (10), um zu erreiche, dass elektrostatische Ladungen in einem Abfüllgerät (100) und der angegliederten Ausrüstung und Pulverpartikel in dem Speicher (110) elektrostatisch neutralisiert werden, so dass der Abfüllvorgang nicht nachteilig beeinflusst wird.

25. Eine elektrodynamische Beladung einer dosierten Dosis (131) einer Trockenpulvermedikamentzubereitung in einen ausgewählten Typ von Behälter (130), wobei die Dosis für einen ausgewählten Pulverinhalator vorgesehen ist, **gekennzeichnet durch**
- Auswählen der Zubereitung, die geladen werden soll, so dass sie mindestens einen pharmakologisch aktiven, trockenen, fein zerteilten Inhaltsstoff, der einen mittleren Partikeldurchmesser von nicht weniger als 0,5 µm und nicht mehr als 6 µm aufweist und optional mindestens einen physiologisch annehmbaren, trockenen, fein zerteilten Hilfsstoff enthält;
- Kontrollieren der Umgebungsbedingungen für die Zubereitung während der Ladung der dosierten Dosis, so dass die relative Feuchtigkeit unter 30%, bevorzugt unter 20%, und am meisten bevorzugt unter 10% gehalten wird;
- Abgabe von Partikeln der Zubereitung auf ein Dosierbett (132), das Teil des ausgewählten Behälters (130) ist, unter Verwendung eines elektrischen Felddosierverfahrens bis eine vorgesehene vordosierte Masse und korrekte Porosität der Dosis (131) erreicht ist;
- Anordnen der Ladung, so dass die Kontur der Dosis (131) eine vordefinierte Geometrie aufweist, die geeignet ist für eine anhaltende Dosisabgabe unter Verwendung eines ausgewählten Pulverinhalators.

26. Ladung nach Anspruch 25, **dadurch gekennzeichnet, dass**
- von dem mindestens einen aktiven Inhaltsstoff mindestens 80 Masse-% und bevorzugt mindestens 90 Masse-%.der Partikel in einem aerodynamischen Durchmesserbereich von 0,1 bis 10 µm und bevorzugter von 0,1 bis 5 µm, und am meisten bevorzugt von 0,1 bis 3 µm liegen, wobei der letztgenannte Bereich insbesondere für systemisch wirkende aktive Inhaltsstoffe wünschenswert ist.

27. Ladung nach Anspruch 25, **gekennzeichnet durch**
- Auswählen des Portionsbehälters (130), so dass er ein Hochleistungssperrschichtsiegelbehälter ist, und
- Versiegeln des Portionsbehälters (130) mit einer Hochleistungssperrschichtsiegelfolie, so dass die Dosis (131) in einer dicht versiegelten Packung eingeschlossen ist, um die Dosis (131) für eine spezifische Zeit vor normalen Veränderungen unter Umgebungsbedingungen unbeeinflusst zu erhalten.

28. Ladung nach Anspruch 25, **gekennzeichnet durch**
- das Kontrollieren der Abscheidung von Pulverpartikeln, so dass die Partikel, die an dem elektrischen Felddosierverfahren beteiligt sind, sich nicht außerhalb des Behälters mit starken Sperreigenschaften (130) verteilen können, wobei die Partikel an dem Kontaminieren der Siegeloberflächen des Behälters vor dem Versiegelungsschritt gehindert werden.

29. Ladung nach Anspruch 25, **gekennzeichnet durch** die Herstellung einer dosierten Dosis (131) der Zubereitung mit einer Masse in einem Bereich von 0,1 bis 50 mg und bevorzugt in einem Bereich von 0,5 bis 25 mg.

30. Ladung nach Anspruch 25, **gekennzeichnet durch**
- Auswählen eines Pulverinhalators, der eine Luftzerteilvorrichtung enthält, zur Abgabe einer ausgewählten, vordosierten Dosis, wobei eine ausgestoßene feine Partikeldosis aus mindestens 30 Masse-% und bevorzugt mehr als 40 Masse-% und am meisten bevorzugt mehr als 50 Masse-% des gesamten pharmakologisch aktiven Inhaltsstoffes der dosierten Dosis besteht.

31. Ladung nach Anspruch 25, **gekennzeichnet durch**
- Auswählen einer Pulverinhalationsvorrichtung, die an Portionsbehälter (130) in Form von Blistern, die mit einer abziehbaren Folie versiegelt sind, angepasst ist, wobei die Folie vor der Verabreichung einer ausgewählten, dosierten Dosis (131) an einen Anwender, der **durch** die Inhalationsvorrichtung inhaliert, abgezogen wird.

32. Ladung nach Anspruch 25, **gekennzeichnet durch**
- Anbringen von Quellen von elektrischen Ladungen, bevorzugt Ionenquellen, in einem Arbeitsabstand zu dem Dosierbett (132) und optional in einem Arbeitsabstand zu dem Pulver (1) in dem Bulkpulverspeicher (110) um zu erreichen, dass die elektrischen Ladungen an den Geräten, die mit dem Dosierprozess und den Pulverpartikeln in dem Speicher in Beziehung sind, elektrisch neutralisiert werden, so dass das Dosierverfahren nicht nachteilig beeinflusst wird.

## Revendications

1. Préparation d'un médicament en poudre sèche comprenant au moins un ingrédient actif sur le plan pharmaceutique, la préparation étant conçue pour une aérosolisation par un inhalateur de poudre sèche, **caractérisée en ce que**
ledit au moins un ingrédient actif est présenté ayant un diamètre de particule moyen de pas moins de 0,5 µm et de pas plus de 6 µm ;
la préparation est adaptée pour une utilisation dans un procédé de formation où la préparation est mesurée et, par force douce, rendue sous forme de charge poreuse (131) non-pulvérulente de particules individuelles non-agglomérées mais cohérentes, et
une ou plusieurs charge(s) poreuse(s) (131) de la préparation, chargée dans un conteneur de dose (130), constituent une dose médicamenteuse mesurée adaptée pour une libération prolongée de dose utilisant l'inhalateur de poudre sèche.

2. Préparation selon la revendication 1, **caractérisée en ce que**
au moins un excipient acceptable sur le plan biologique est compris dans la préparation.

3. Préparation selon la revendication 1, **caractérisée en ce que**
la ou les charge(s) poreuse(s) (131), constituant la dose mesurée, est/sont ajustée(s) jusqu'à une désagrégation et une dispersion progressives dans un courant d'air d'inhalation résultant d'un acte d'inhalation réalisé en utilisant l'inhalateur à poudre sèche.

4. Préparation selon la revendication 1, **caractérisée en ce que**
ledit au moins un ingrédient actif présente au moins 80 % et de préférence au moins 90 % en masse de particules dans une plage de diamètre aérodynamique allant de 0,1 à 10 µm, et de façon plus préférée de 0,1 à 5 µm, et de façon particulièrement préférée de 0,1 à 3 µm, cette dernière plage étant particulièrement souhaitable pour les ingrédients actifs agissant de façon systémique.

5. Préparation selon la revendication 1, **caractérisée en ce que**
un ingrédient pharmacologique actif nominal ciblé génère une dose pré-mesurée de la préparation, située dans une plage allant de 0,1 à 50 mg, et de préférence dans une plage allant de 0,5 à 25 mg.

6. Préparation selon la revendication 1, **caractérisée en ce que**
le conteneur de dose (130) a d'importantes propriétés de barrière d'étanchéité, ce qui le rend imperméable à l'humidité et aux autres corps étrangers.

7. Préparation selon la revendication 1, **caractérisée en ce que**
une dose de la préparation est mesurée et chargée sur le lit de dose (132) faisant partie d'un conteneur à haute étanchéité (130) à des conditions ambiantes avec une humidité relative inférieure à 30 %, de préférence inférieure à 20 %, et de façon particulièrement préférée inférieure à 10 %.

8. Préparation selon la revendication 1, **caractérisée en ce que**
le procédé de formation est choisi pour être un procédé de dosage par champ électrique (ELFID) employant un procédé consistant à utiliser des champs électriques et des particules de la préparation médicamenteuse chargées électriquement pour former une dose pré-mesurée (131) directement sur un lit de dose (132) faisant partie d'un type de conteneur de dose (130) choisi.

9. Préparation selon la revendication 1, **caractérisée en ce que**
le procédé de formation est choisi pour être un procédé volumétrique utilisant la gravité et éventuellement l'énergie électrique, mécanique ou pneumatique pour mesurer et remplir des charges (131) de la préparation de médicament, formant ainsi une dose pré-mesurée dans un type de conteneur de dose (130) choisi.

10. Préparation selon la revendication 1, **caractérisée en ce que**
ledit au moins un ingrédient pharmacologique actif est choisi parmi un groupe de substances comprenant la vasopressine, un analogue de la vasopressine, la desmopressine, les peptides de type glucagons, la corticotropine, la gonadotropine, la calcitonine, le peptide C de l'insuline, l'hormone parathyroïde, l'hormone de croissance humaine, l'hormone de croissance, l'hormone de libération de l'hormone de croissance, l'oxytocine, l'hormone de libération de la corticotropine, un analogue de la somatostatine, un analogue de l'agoniste de la gonadotropine, un peptide natriurétique atrial, l'hormone de libération de la thyroxine, l'hormone de stimulation folliculaire, la prolactine, une interleukine, un facteur de croissance, un vaccin polypeptidique, une enzyme, une endorphine, une glycoprotéine, une lipoprotéine, une kinase, des récepteurs intracellulaires, des facteurs de transcription, des activateurs/récepteurs de transcription des gènes, des neurotransmetteurs, des protéoglycanes, un polypeptide impliqué dans la cascade de coagulation sanguine qui exerce son effet pharmacologique de façon systémique, tout autre polypeptide ayant une masse moléculaire (Daltons) allant jusqu'à 200 kDa, des protéines, des polysaccharides, des lipides, des acides nucléiques et des combinaisons de ceux-ci, ou parmi le groupe constitué par le leuprolide et l'albutérol, les opiacés, la nicotine, les dérivés de la nicotine, la scopolamine, la morphine, les analogues de l'apomorphine, le sumatriptan, le rizatriptan, l'almotriptan, l'élétriptan, le frovatriptan, les substances actives pour troubles respiratoires et les sels de celles-ci, tels que le formotérol, le budésonide, l'ipratropium, le fluticasone, le tiotropium, le salbutamol et le mométasone.

11. Préparation selon la revendication 1, **caractérisée en ce que**
ledit au moins un excipient acceptable sur le plan physiologique, sec, finement divisé, est choisi parmi un groupe de substances comprenant le glucose, l'arabinose, le lactose, le monohydrate de lactose, l'anhydride de lactose, le saccharose, le maltose, la dextrane, le sorbitol, le mannitol, le xylitol, le chlorure de sodium, le carbonate de calcium ou les mélanges de ceux-ci.

12. Préparation selon la revendication 1, **caractérisée en ce que**
au moins un excipient solide, acceptable sur le plan physiologique, ayant un diamètre de particule aérodynamique moyen supérieur à 20 µm est éventuellement ajouté à l'étape de mélange, ledit excipient étant choisi parmi un groupe de substances comprenant le glucose, l'arabinose, le lactose, le monohydrate de lactose, l'anhydride de lactose, le saccharose, le maltose, la dextrane, le sorbitol, le mannitol, le xylitol, le chlorure de sodium, le carbonate de calcium ou les mélanges de ceux-ci.

13. Préparation selon la revendication 1, **caractérisée en ce que**
un inhalateur de poudre sèche incorporant un dispositif de rasoir à air est choisi pour émettre une dose choisie, mesurée.

14. Préparation selon la revendication 1, **caractérisée en ce que**
un dispositif d'inhalation de poudre sèche est choisi pour émettre une dose choisie, mesurée, ledit dispositif étant adapté à des conteneurs de dose (130) sous la forme d'emballages-coques scellés par un film pelable, qui sera éliminé en étant pelé avant l'administration de la dose à l'utilisateur inhalant à l'aide du dispositif d'inhalation.

15. Procédé de formation et de chargement d'une dose mesurée volumétriquement (131) d'une préparation de poudre sèche dans un type choisi de conteneur de dose (130), ladite dose étant conçue pour un inhalateur de poudre sèche choisi, **caractérisé par** les étapes consistant à
choisir la préparation de façon à ce qu'elle comprenne au moins un ingrédient actif sur le plan pharmaceutique, sec, finement divisé, ayant un diamètre de particule moyen de pas moins de 0,5 µm et de pas plus de 6 µm ;
ajuster les conditions ambiantes pour la préparation lors de la formation et du chargement d'une dose mesurée, de façon à ce que l'humidité relative soit maintenue en dessous de 30 %, de préférence en-dessous de 20 %, et de façon particulièrement préférée en-dessous de 10 % ;
charger la préparation à partir d'un élément de stockage de poudre en vrac (110) dans au moins un réceptacle de poudre (10) agissant en tant que cavité de mesure ;
appliquer une force légère sur la charge en ajoutant de l'énergie afin de rassembler les particules de poudre pour former une charge poreuse (131) de particules non-agglomérées mais cohérentes dans la cavité de mesure, et
éjecter la charge (131), dont les contours présentent une géométrie définie, dans le type de conteneur (130) choisi, de façon à ce que le corps de la dose ne puisse se désintégrer et former un amas.

16. Procédé de formation selon la revendication 15, **caractérisé par** l'étape supplémentaire consistant à
exiger que ledit au moins un ingrédient actif présente au moins 80 % et de préférence au moins 90 % en masse de particules dans une plage de diamètre aérodynamique allant de 0,1 à 10 µm, et de façon plus préférée de 0,1 à 5 µm, et de façon particulièrement préférée de 0,1 à 3 µm, cette dernière plage étant particulièrement souhaitable pour les ingrédients actifs agissant de façon systémique.

17. Procédé selon la revendication 15, **caractérisé par** l'étape supplémentaire consistant à
forcer légèrement les particules de poudre dans la cavité de mesure de dose (10), obtenant ainsi une charge de dose poreuse (131) de particules cohérentes, de façon à ce que les particules de la charge (131) ne puissent se répandre hors du conteneur (130) lorsque la charge (131) est éjectée, empêchant ainsi les particules de contaminer les surfaces de scellage du conteneur avant une étape de scellage.

18. Procédé selon la revendication 15, **caractérisé par** l'étape supplémentaire consistant à
produire une dose mesurée de la préparation, avec une masse située dans une plage allant de 0,1 à 50 mg, et de préférence dans une plage allant de 0,5 à 25 mg.

19. Procédé selon la revendication 15, **caractérisé par** l'étape supplémentaire consistant à
choisir un inhalateur de poudre sèche incorporant un dispositif de rasoir à air pour administrer une dose choisie, mesurée à un utilisateur, une dose de fines particules administrée constituant en masse au moins 30 %, et de préférence plus de 40 %, et de façon particulièrement préférée 50 % de l'ingrédient actif sur le plan pharmacologique total.

20. Procédé selon la revendication 15, **caractérisé par** l'étape supplémentaire consistant à
choisir un dispositif d'inhalation de poudre sèche assurant une libération prolongée d'une dose mesurée, choisie, à un utilisateur.

21. Procédé selon la revendication 15, **caractérisé par** l'étape supplémentaire consistant à
choisir un dispositif d'inhalation de poudre sèche adapté à des conteneurs de dose sous la forme d'emballages-coques scellés par un film pelable, qui sera éliminé en étant pelé avant l'administration d'une dose mesurée choisie à un utilisateur inhalant à l'aide du dispositif d'inhalation.

22. Procédé selon la revendication 15, **caractérisé par** l'étape supplémentaire consistant à
utiliser un conteneur de dose (130) ayant d'importantes propriétés de barrière d'étanchéité, qui empêchera la dose d'être affectée par les changements normaux de conditions ambiantes pendant une durée spécifiée de stockage avant l'utilisation, et pendant une durée d'utilisation spécifiée.

23. Procédé selon la revendication 15, **caractérisé par** l'étape supplémentaire consistant à
utiliser une force d'aspiration (114) et de l'air sous pression (115) en tant qu'énergie de force ajoutée lors de la formation de la charge (131).

24. Procédé selon la revendication 15, **caractérisé par** l'étape supplémentaire consistant à
disposer des sources de charges électriques, de préférence des sources ioniques, à une distance de travail par rapport au réceptacle de dose (10) et éventuellement à une distance de travail par rapport à la poudre (1) dans l'élément de stockage de poudre en vrac (110) afin de garantir que les charges électrostatiques sur un outil de remplissage (100) et les équipements associés et les particules de poudre dans l'élément de stockage (110) deviennent neutralisées sur le plan électrique de façon à ce que le processus de remplissage ne soit pas affecté de façon nuisible.

25. Chargement électrodynamique d'une dose mesurée (131) d'une préparation de médicament en poudre sèche dans un type de conteneur (130) choisi, ladite dose étant conçue pour un inhalateur de poudre sèche choisi, **caractérisé par** les étapes consistant à
choisir la préparation à charger de façon à ce qu'elle comprenne au moins un ingrédient actif sur le plan pharmaceutique, sec, finement divisé, ayant un diamètre de particule moyen de pas moins de 0,5 µm et de pas plus de 6 µm, et éventuellement au moins un excipient acceptable sur le plan physiologique, sec, finement divisé ;
contrôler les conditions ambiantes pour la préparation lors du chargement de la dose mesurée, de façon à ce que l'humidité relative soit maintenue en dessous de 30 %, de préférence en-dessous de 20 %, et de façon particulièrement préférée en-dessous de 10 % ;
déposer des particules de la préparation sur un lit de dose (132), faisant parti du conteneur (130) choisi, en utilisant un procédé de dosage par champ électrique jusqu'à l'obtention de la masse pré-mesurée et de la porosité voulues de la dose (131) ;
régler le chargement de façon à ce que les contours de la dose (131) présentent une géométrie prédéfinie, adaptée à une libération de dose prolongée en utilisant l'inhalateur de poudre sèche choisi.

26. Chargement selon la revendication 25, **caractérisé en ce que**
ledit au moins un ingrédient actif présente au moins 80 % et de préférence au moins 90 % en masse de particules dans une plage de diamètre aérodynamique allant de 0,1 à 10 µm, et de façon plus préférée de 0,1 à 5 µm, et de façon particulièrement préférée de 0,1 à 3 µm, cette dernière plage étant particulièrement souhaitable pour les ingrédients actifs agissant de façon systémique.

27. Chargement selon la revendication 25, **caractérisé par** les étapes consistant à
choisir le conteneur de dose (130) de façon à ce qu'il soit un conteneur à haute barrière d'étanchéité, et
sceller le conteneur de dose (130) avec un film de scellage à haute barrière d'étanchéité, enfermant ainsi la dose (131) dans un emballage étroitement scellé afin d'empêcher que la dose (131) soit affectée par les changements normaux de conditions ambiantes pendant une durée spécifiée.

28. Chargement selon la revendication 25, **caractérisé par** l'étape consistant à
contrôler le dépôt de particules de poudre de façon à ce que les particules impliquées dans le processus de dosage par champ électrique ne puissent se répandre hors du conteneur à haute barrière d'étanchéité (130), empêchant ainsi les particules de contaminer les surfaces de scellage du conteneur avant une étape de scellage.

29. Chargement selon la revendication 25, **caractérisé par** l'étape consistant à
produire une dose mesurée (131) de la préparation, avec une plage de masse allant de 0,1 à 50 mg, et de préférence une plage de masse allant de 0,5 à 25 mg.

30. Chargement selon la revendication 25, **caractérisé par** l'étape consistant à
choisir un inhalateur de poudre sèche incorporant un dispositif de rasoir à air pour libérer une dose choisie, pré-mesurée, une dose de particules fines émises constituant en masse au moins 30 %, et de préférence plus de 40 % et de façon particulièrement préférée plus de 50 % de l'ingrédient actif sur le plan pharmacologique total de la dose mesurée.

31. Chargement selon la revendication 25, **caractérisé par** l'étape consistant à
choisir un inhalateur de poudre sèche adapté à des conteneurs de dose (130) sous la forme d'emballages-coques scellés par un film pelable, qui sera éliminé en étant pelé avant l'administration d'une dose mesurée choisie (131) à l'utilisateur inhalant à l'aide du dispositif d'inhalation.

32. Chargement selon la revendication 25, **caractérisé par** l'étape consistant à
disposer des sources de charges électriques, de préférence des sources ioniques, à une distance de travail par rapport au lit de dose (132) et éventuellement à une distance de travail par rapport à la poudre (1) dans l'élément de stockage de poudre en vrac (110) afin de garantir que les charges électrostatiques sur les équipements associés au processus de dosage et les particules de poudre dans l'élément de stockage deviennent neutralisées sur le plan électrique, de façon à ce que le processus de dosage ne soit pas affecté de façon nuisible.
